# EUROPEAN PATENT APPLICATION

(11) **EP 4 610 306 A1**
(43) Date of publication of application: **03.09.2025**
(21) Application number: 24160328.1
(22) Date of filing: 28.02.2024
(51) Int. Cl.: C08L 25/10, C08L 53/00, A61M 5/315, C08K 3/34

(54) **LOW PROCESSING TEMPERATURE ELASTOMERS**

(71) Applicant: Becton, Dickinson and Company, Franklin Lakes, NJ 07417-1880 (US)
(72) Inventor: RODRIGUEZ, Gumersindo, Sumter, SC29154 (US); FORCINITI, Leandro, 410 Wharton, NJ07885 (US); RODRIGUEZ SAN JUAN, Nestor, Hamburg, NJ07419 (US)
(74) Representative: Germain Maureau

(57) **Abstract**

The invention relates, in general, to various elastomeric, polymeric and/or rubber formulations that can be processed at low temperatures, methods of making same, and uses for such compounds. In one embodiment, the present invention relates to an elastomeric, polymeric and/or rubber formulation that can be processed at a low temperatures where such elastomeric, polymeric and/or rubber formulations are formed from one or more suitable elastomers, polymers and/or rubbers such as one or more crosslinked backbone unsaturated styrene terpolymers. The elastomeric, polymeric and/or rubber formulations of the present invention are, in one embodiment, able to be quickly processed at a suitably low temperature of less than about 130 °C and therefore are suitable for a wide range of applications including, but not limited to, various medical applications such as various hermitically sealed applications.

## Description

### BACKGROUND OF THE INVENTION

### Field of the Invention

The invention relates, in general, to various elastomeric, polymeric and/or rubber formulations that can be processed at low temperatures, methods of making same, and uses for such compounds. In one embodiment, the present invention relates to an elastomeric, polymeric and/or rubber formulation that can be processed at a low temperatures where such elastomeric, polymeric and/or rubber formulations are formed from one or more suitable elastomers, polymers and/or rubbers such as one or more crosslinked backbone unsaturated styrene terpolymers. The elastomeric, polymeric and/or rubber formulations of the present invention are, in one embodiment, able to be quickly processed at a suitably low temperature of less than about 130 °C and therefore are suitable for a wide range of applications including, but not limited to, various medical applications such as various hermitically sealed applications.

### Description of the Related Art

Various rubber compositions, compounded rubbers, and/or rubber compounds (hereinafter referred to just as "rubber compositions") are known and used as closure materials in containers such as prefilled syringes and other medical applications. However, to date such rubber compositions suffer from one or more disadvantages including, but not limited to, high vulcanization, or even processing, temperatures (generally above 175 °C, or even 200 °C), require silicone oil lubrication, require high break-out forces to function properly, and/or do not glide easily, or readily, over a wide range of surface interfaces with various glasses and/or plastics.

As such, there is a need in the art for a rubber composition that is able to be processed at a low curing temperature (generally lower than about 130 °C), does not require the use of a silicone oil lubrication, are able to operate properly at lower break-out forces, and/or glide easily, or readily, over a wide range of surface interfaces with various glasses and/or plastics.

Furthermore, there is a need in the art for a rubber composition that is compatible with various lamination processes to yield a partially, or fully, laminated sealing device, or in particular a partially, or fully, laminated stopper for medical applications.

### SUMMARY OF THE INVENTION

The invention relates, in general, to various elastomeric, polymeric and/or rubber formulations that can be processed at low temperatures, methods of making same, and uses for such compounds. In one embodiment, the present invention relates to an elastomeric, polymeric and/or rubber formulation that can be processed at a low temperatures where such elastomeric, polymeric and/or rubber formulations are formed from one or more suitable elastomers, polymers and/or rubbers such as one or more crosslinked backbone unsaturated styrene terpolymers. The elastomeric, polymeric and/or rubber formulations of the present invention are, in one embodiment, able to be quickly processed at a suitably low temperature of less than about 130 °C and therefore are suitable for a wide range of applications including, but not limited to, various medical applications such as various hermitically sealed applications.

According to one aspect, the present invention is directed to a polymer material comprising: at least one polymer compound having a Young's modulus in the range of about 5 MPa to about 15 MPa and a Shore A hardness in the range of about 60 to about 100, wherein the polymer material is processed at a temperature in the range of about 90 °C to about 130 °C. In one embodiment, the processing of the polymer material is defined as an increase in ultimate torque response as measured by cure rheometer (ASTM 05289) of at least 400 percent within 10 minutes of isothermal conditioning at a platen temperature between about 90 °C and about 130 °C. In another embodiment, the polymer material of the present invention is formed from a polymer compound having a Young's modulus in the range of about 7.5 MPa to about 12.5 MPa and a Shore A hardness in the range of about 70 to about 90. In still another embodiment, the polymer material of the present invention is selected from one or more styrene terpolymers. In still another embodiment, the one or more styrene terpolymers are selected from one or more crosslinked backbone unsaturated styrene terpolymers. In still another embodiment, the one or more styrene terpolymers are selected from at least one brominated isobutylene paramethyl-styrene terpolymers (BIMSM) rubber with no unsaturation on the backbone of the polymer. In still another embodiment, the polymer material of the present invention further comprises from about 50 phr to about 100 phr (parts per hundred rubber) of at least one non-staining halogenated isobutylene-isoprene copolymer and/or brominated isobutylene paramethyl-styrene terpolymers (BIMSM) of low to high Mooney viscosity. In still another embodiment, the polymer material of the present invention further comprises about 50 phr to about 70 phr of calcined magnesium silicate. In still another embodiment, the polymer material of the present invention further comprises about 10 phr to about 150 phr of at least one silica filler. In still another embodiment, the at least one silica filler is modified with at least one coupling agent. In still another embodiment, the polymer material of the present invention further comprises up to about 50 phr of at least one processing oil.

In another embodiment, the present invention is directed to a stopper comprising: a stopper body formed from any of the elastomeric, polymeric and/or rubber materials disclosed herein; and at least one sealing rib formed on at least one portion of the stopper body. In still another embodiment, the stopper body further comprises at least two sealing ribs formed thereon. In still another embodiment, the at least one of the sealing ribs is laminated with at least one lamination layer. In still another embodiment, all of the sealing ribs are laminated with at least one lamination layer. In still another embodiment, all of the sealing ribs and the stopper body are laminated with at least one lamination layer. In still another embodiment, the at least one lamination layer is formed from at least one compound selected form a polyethylene such as a UHMWPE, copolymers thereof, and combinations thereof. In still another embodiment, the at least one lamination layer is formed from at least one layer of UHMWPE.

In still another embodiment, the present invention is directed to a syringe comprising: a syringe barrel; a plunger, wherein the plunger is designed to fit within the syringe barrel; and a stopper according to any of the embodiments disclosed herein and formed from any of the elastomeric, polymeric and/or rubber materials disclosed herein, wherein the stopper is adapted for attachment to one end of the plunger so as act as a seal for the syringe barrel.

In still another embodiment, the present invention is directed to a sealing device comprising: a sealing body formed from any of the elastomeric, polymeric and/or rubber materials disclosed herein; and at least one sealing structure and/or or sealing rib formed on at least one portion of the sealing body, that can be utilized to seal any type of medical device and/or other device needing an adequate sealing device.

Clause 1: A polymer material comprising: at least one polymer compound having a Young's modulus in the range of about 5 MPa to about 15 MPa and a Shore A hardness in the range of about 60 to about 100, wherein the polymer material is processed at a temperature in the range of about 90 °C to about 130 °C.

Clause 2: The polymer material of clause 1, wherein the processing of the polymer material is defined as an increase in ultimate torque response as measured by cure rheometer (ASTM 05289) of at least 400 percent within 10 minutes of isothermal conditioning at a platen temperature between about 90 °C and about 130 °C.

Clause 3: The polymer material of any of clauses 1 or 2, wherein at least one polymer compound having a Young's modulus in the range of about 7.5 MPa to about 12.5 MPa and a Shore A hardness in the range of about 70 to about 90.

Clause 4: The polymer material of any of clauses 1 to 3, wherein the at least one polymer compound is selected from one or more styrene terpolymers.

Clause 5: The polymer material of clause 4, wherein the one or more styrene terpolymers are selected from one or more crosslinked backbone unsaturated styrene terpolymers.

Clause 6: The polymer material of clause 4, wherein the one or more styrene terpolymers are selected from at least one brominated isobutylene paramethyl-styrene terpolymers (BIMSM) rubber with no unsaturation on the backbone of the polymer.

Clause 7: The polymer material of any of clauses 1 to 6, wherein the polymer material further comprises from about 50 phr to about 100 phr of at least one non-staining halogenated isobutylene-isoprene copolymer and/or brominated isobutylene paramethyl-styrene terpolymers (BIMSM) of low to high Mooney viscosity.

Clause 8: The polymer material of any of clauses 1 to 7, wherein the polymer material further comprises about 50 phr to about 70 phr of calcined magnesium silicate.

Clause 9: The polymer material of any of clauses 1 to 8, wherein the polymer material further comprises about 10 phr to about 150 phr of at least one silica filler.

Clause 10: The polymer material of clause 9, wherein the at least one silica filler is modified with at least one coupling agent.

Clause 11: The polymer material of any of clauses 1 to 10, wherein the polymer material further comprises up to about 50 phr of at least one processing oil.

Clause 12: A stopper comprising: a stopper body formed from a polymeric material of any of clauses 1 to 11; and at least one sealing rib formed on at least one portion of the stopper body.

Clause 13: The stopper of clause 12, wherein the stopper body further comprises at least two sealing ribs formed thereon.

Clause 14: The stopper of any of clauses 12 or 13, wherein at least one of the sealing ribs is laminated with at least one lamination layer.

Clause 15: The stopper of any of clauses 12 to 14, wherein all of the sealing ribs are laminated with at least one lamination layer.

Clause 16: The stopper of any of clauses 12 to 15, wherein all of the sealing ribs and the stopper body are laminated with at least one lamination layer.

Clause 17: The stopper of any of clauses 12 to 16, wherein the at least one lamination layer is formed from at least one compound selected form a polyethylene such as a UHMWPE, copolymers thereof, and combinations thereof.

Clause 18: The stopper of clause 17, wherein the at least one lamination layer is formed from at least one layer of UHMWPE.

Clause 19: A syringe comprising: a syringe barrel; a plunger, wherein the plunger is designed to fit within the syringe barrel; and a stopper according to any of clauses 12 to 18, wherein the stopper is adapted for attachment to one end of the plunger so as act as a seal for the syringe barrel.

Clause 20: A sealing device comprising: a sealing body formed from a polymeric material of any of clauses 1 to 11; and at least one sealing structure and/or or sealing rib formed on at least one portion of the sealing body.

### BRIEF DESCRIPTION OF THE DRAWINGS

Figure 1 is perspective view of an exemplary syringe with which embodiments of the disclosure may be implemented;
Figure 2 is a perspective view of the syringe of Figure 1 showing a needle shield covering a distal end of the syringe;
Figure 3 is a perspective view of a stopper according to a first embodiment of the present invention;
Figure 4 is a cross-sectional view of one stopper design according to one embodiment of the present invention where the stopper contains an Acme thread therein;
Figure 5 is a cross-sectional view of one stopper design according to another embodiment of the present invention where the stopper contains a snap-in plunger rod connector therein;
Figure 6 is a cross-sectional view of one stopper design according to still another embodiment of the present invention where the stopper contains a buttress tread therein;
Figure 7 is a cross-sectional view of one stopper design according to still another embodiment of the present invention where the stopper contains a buttress tread therein; and
Figure 8 is a cross-sectional view of one stopper design according to still another embodiment of the present invention where the stopper contains a buttress tread therein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As noted above, the invention relates, in general, to various elastomeric, polymeric and/or rubber formulations that can be processed at low temperatures, methods of making same, and uses for such compounds. In one embodiment, the present invention relates to an elastomeric, polymeric and/or rubber formulation that can be processed at a low temperatures where such elastomeric, polymeric and/or rubber formulations are formed from one or more suitable elastomers, polymers and/or rubbers such as one or more crosslinked backbone unsaturated styrene terpolymers. The elastomeric, polymeric and/or rubber formulations of the present invention are, in one embodiment, able to be quickly processed at a suitably low temperature of less than about 130 °C and therefore are suitable for a wide range of applications including, but not limited to, various medical applications such as various hermitically sealed applications.

Initially, a discussion of one set of exemplary stopper designs will be undertaken. However, it is to be understood that the following stopper design is only representative and the present invention is not limited to solely the designs disclosed herein. Rather, any stopper design where total sealing is desired can be utilized in conjunction with the present invention.

For purposes of the description hereinafter, the terms "upper", "lower", "right", "left", "vertical", "horizontal", "top", "bottom", "lateral", "longitudinal", and derivatives thereof, shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

As used herein, the terms "rubber composition", "rubber material", "compounded rubber", "compounded rubber material", "rubber compound", "polymer composition", "polymer material", "compounded polymer", "compounded polymer material", and "polymer compound" are used interchangeably to refer to a polymer/rubber which has been blended or mixed with one or more various ingredients and/or materials as described herein.

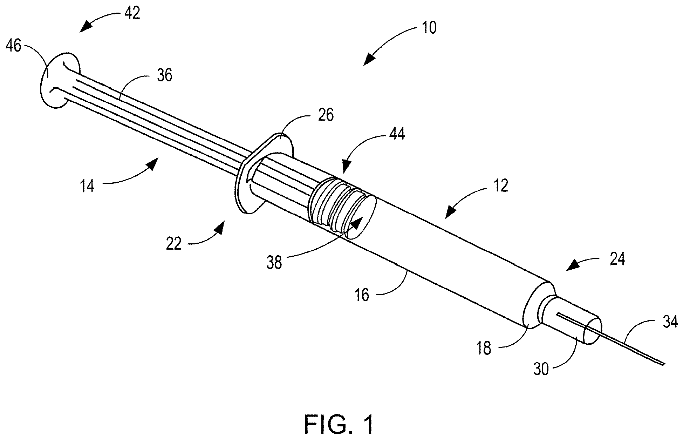

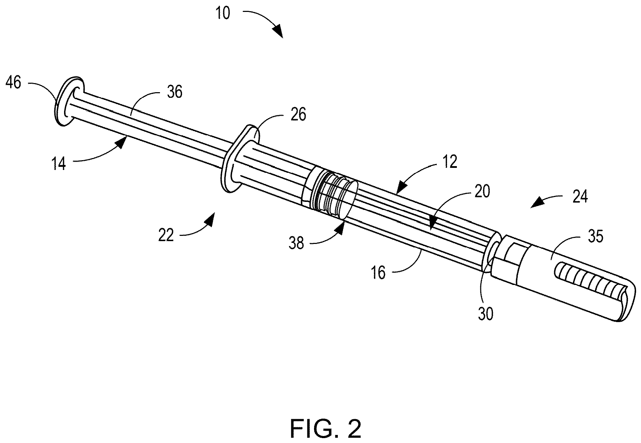

Referring to Figures 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in to Figures 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed from a suitable glass or plastic material and includes a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a hub 30 which narrows with respect to the cylindrical outer wall 16 and extends out distally therefrom. The hub portion 30 is formed as a partially hollow member that defines a channel therethrough in fluid communication with the chamber 20. A needle 34 is attached to the hub portion 30 within the channel, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. A thumb press 46 is positioned at the plunger proximal end 42 that may be engaged by a thumb (or other finger) of the user to apply a distally directed force to the plunger assembly 14 to move the plunger 36 with respect to the syringe barrel 12. The plunger distal end 44 is configured to mate with the stopper 38. In other embodiments, the plunger distal end 44 may be a threaded end that mates with the stopper 38.

The stopper 38 of plunger assembly 14 is positioned at the plunger distal end 44 so as to be movable along with the plunger 36 within the chamber 20 of syringe barrel 12. That is, as a distally-directed force is applied to the plunger rod 36 (such as to thumb press 46), both the plunger rod 36 and the stopper 38 are caused to move distally through syringe barrel 12, with the stopper 38 configured to maintain a seal with an inner surface of the syringe barrel 12 as it is moved therethrough.

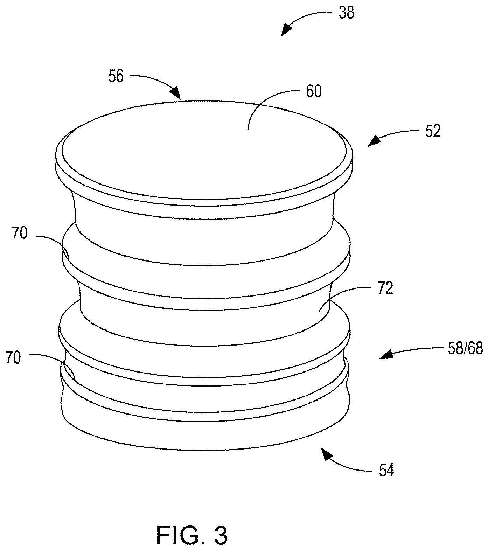

Referring now to Figure 3, and with continued reference to Figures 1 and 2, the structure of an exemplary stopper 38 that may be included in syringe 10 is shown in greater detail, according to a non-limiting aspect or embodiment of the disclosure. The stopper 38 is defined by a main body 52 that includes a proximal end 54 and a distal end 56. The proximal end 54 may be configured as an open proximal end that engages with the distal end of plunger 36, while the distal end 56 is configured as a closed distal end configured to engage with the barrel 12 of syringe 10. The closed distal end 56 of the main body 52 includes a generally cylindrical portion 58 and a roof portion 60. In some embodiments, the roof portion 60 is configured as a flat surface to provide the closed distal end 56, but it is recognized that the roof portion 60 may instead be configured as a conical or domed surface, as other non-limiting examples.

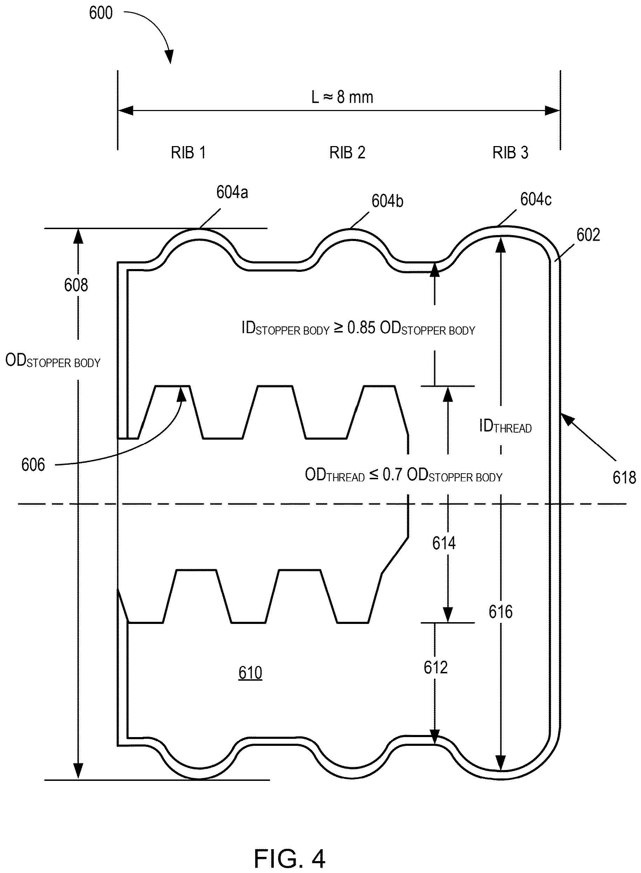

As known in the art, the main body 52 of the stopper 38 is partially hollow and is sized and configured to receive the distal end 44 of plunger 36 therein. The main body 52 of the stopper 38 defines an inner cavity (visible in Figures 4-8) that, in some embodiments, may include a threaded inner surface that is configured to receive and mate with a threaded distal end 44 of plunger 36. In other embodiments, the inner cavity may define an inner contoured surface having a notch therein that is configured to receive a flanged extension member of plunger 36.

The outer surface 68 of the cylindrical portion 58 of the main body 52 includes one or more ribs thereon that extend circumferentially around the entire outer surface 68 of the cylindrical portion 58. In each of the illustrated embodiments, a plurality of ribs 70 are included on the stopper 38, but it is recognized that other embodiments could include only a single rib 70. The ribs 70 may be formed integrally with the main body 52, such as via a molding process. The plurality of ribs 70 may include two ribs 70, with a first or distal rib positioned toward the distal end 56 of the main body 52 and a second or proximal rib positioned toward the proximal end 54 of the main body 52, or may include three or more ribs 70 spaced apart along the cylindrical portion 58 - with an inter-rib region 72 provided between each adjacent pair of ribs. The inter-rib region 72 of cylindrical portion 58 may have a generally flat outer surface 68 or a curved outer surface 68.

The stopper 38 may be made from a material that is different from the material of the plunger 36 and that is capable of forming a tight seal with the syringe barrel 12 as it is advanced therethrough. In some embodiments, the stopper 38 is desirably manufactured from any suitable elastomeric, polymeric and/or rubber (hereinafter referred to inclusively as solely a "polymeric material" for brevity's sake) material with a Young's modulus (also known as the tensile modulus, elastic modulus or traction modulus) in a range of about 5 MPa to about 15 MPa, or from about 5.25 MPa to about 14.75 MPa, or from about 5.5 MPa to about 14.5 MPa, or from about 5.75 MPa to about 14.25 MPa, or from about 6 MPa to about 14 MPa, or from about 6.25 MPa to about 13.75 MPa, or from about 6.5 MPa to about 13.5 MPa, or from about 6.75 MPa to about 13.25 MPa, or from about 7 MPa to about 13 MPa, or from about 7.25 MPa to about 12.75 MPa, or from about 7.5 MPa to about 12.5 MPa, or from about 7.75 MPa to about 12.25 MPa, or from about 8 MPa to about 12 MPa, or from about 8.25 MPa to about 11.75 MPa, or from about 8.5 MPa to about 11.5 MPa, or from about 8.75 MPa to about 11.25 MPa, or from about 9 MPa to about 11 MPa, or from about 9.25 MPa to about 10.75 MPa, or from about 9.5 MPa to about 10.5 MPa, or from about 9.75 MPa to about 10.25 MPa, or even about 10 MPa. In another embodiment, stopper 38 is desirably manufactured from any suitable polymeric material with a Young's modulus (also known as the tensile modulus, elastic modulus or traction modulus) in a range of about 5 MPa to about 15 MPa, or from about 5.1 MPa to about 14.9 MPa, or from about 5.2 MPa to about 14.8 MPa, or from about 5.3 MPa to about 14.7 MPa, or from about 5.4 MPa to about 14.6 MPa, or from about 5.5 MPa to about 14.5 MPa, or from about 5.6 MPa to about 14.4 MPa, or from about 5.7 MPa to about 14.3 MPa, or from about 5.8 MPa to about 14.2 MPa, or from about 5.9 MPa to about 14.1 MPa, or from about 6 MPa to about 14 MPa, or from about 6.1 MPa to about 13.9 MPa, or from about 6.2 MPa to about 13.8 MPa, or from about 6.3 MPa to about 13.7 MPa, or from about 6.4 MPa to about 13.6 MPa, or from about 6.5 MPa to about 13.5 MPa, or from about 6.6 MPa to about 13.4 MPa, or from about 6.7 MPa to about 13.3 MPa, or from about 6.8 MPa to about 13.2 MPa, or from about 6.9 MPa to about 13.1 MPa, or from about 7 MPa to about 13 MPa, or from about 7.1 MPa to about 12.9 MPa, or from about 7.2 MPa to about 12.8 MPa, or from about 7.3 MPa to about 12.7 MPa, or from about 7.4 MPa to about 12.6 MPa, or from about 7.5 MPa to about 12.5 MPa, or from about 7.6 MPa to about 12.4 MPa, or from about 7.7 MPa to about 12.3 MPa, or from about 7.8 MPa to about 12.2 MPa, or from about 7.9 MPa to about 12.1 MPa, or from about 8 MPa to about 12 MPa, or from about 8.1 MPa to about 11.9 MPa, or from about 8.2 MPa to about 11.8 MPa, or from about 8.3 MPa to about 11.7 MPa, or from about 8.4 MPa to about 11.6 MPa, or from about 8.5 MPa to about 11.5 MPa, or from about 8.6 MPa to about 11.4 MPa, or from about 8.7 MPa to about 11.3 MPa, or from about 8.8 MPa to about 11.2 MPa, or from about 8.9 MPa to about 11.1 MPa, or from about 9 MPa to about 11 MPa, or from about 9.1 MPa to about 10.9 MPa, or from about 9.2 MPa to about 10.8 MPa, or from about 9.3 MPa to about 10.7 MPa, or from about 9.4 MPa to about 10.6 MPa, or from about 9.5 MPa to about 10.5 MPa, or from about 9.6 MPa to about 10.4 MPa, or from about 9.7 MPa to about 10.3 MPa, or from about 9.8 MPa to about 10.2 MPa, or from about 9.9 MPa to about 10.1 MPa, or even about 10 MPa. Here, as well as elsewhere in the specification and claims, individual numerical values can be combined to form additional, or even new/non-disclosed, numerical ranges.

The desired Shore A hardness of the afore-mentioned suitable polymer materials for stopper 38 of the present invention is between about 60 to about 120, or from about 65 to about 115, or from about 70 to about 110, or from about 75 to about 105, or from about 80 to about 100, or from about 85 to about 95, or even about 90. In another embodiment, stopper 38 is desirably manufactured from any suitable polymeric material with a Shore A hardness of about 60 to about 120, or from about 62 to about 118, or from about 64 to about 116, or from about 66 to about 114, or from about 68 to about 112, or from about 70 to about 110, or from about 72 to about 108, or from about 74 to about 106, or from about 76 to about 104, or from about 78 to about 102, or from about 80 to about 100, or from about 82 to about 98, or from about 84 to about 96, or from about 86 to about 94, or from about 88 to about 92, or even about 90. Here, as well as elsewhere in the specification and claims, individual numerical values can be combined to form additional, or even new/non-disclosed, numerical ranges.

Suitable stopper polymeric materials include, but are not limited to, one or more polyolefins (e.g., PE, PP, and their copolymers), one or more polyamides (e.g., nylons), one or more polyesters (e.g., PET), one or more polystyrenes, one or more polyurethanes, one or more polycarbonates, one or more acrylonitrile-butadiene-styrenes, one or more polyacrylates, one or more elastomeric rubbers, one or more styrene terpolymers, or blends of any two or more thereof that meet at least one of the Young's modulus and/or Shore A properties defined above. In another embodiment, suitable stopper materials include, but are not limited to, polymeric materials selected from one or more polyisoprenes (including, but not limited to, natural rubber), one or more polyisobutylenes, one or more synthetic polyisoprenes, one or more polybutadienes (commonly referred to collectively as butadiene rubbers), one or more chloroprene rubbers (e.g., polychloroprene, neoprene, etc.), one or more butyl rubbers (i.e., copolymers of isobutene and isoprene), one or more halogenated butyl rubbers (e.g., chloro butyl rubber or bromo butyl rubber), one or more styrene-butadiene rubbers (i.e., copolymers of styrene and butadiene), one or more nitrile rubbers (i.e., copolymers of butadiene and acrylonitrile), one or more hydrogenated nitrile rubbers, natural rubber, one or more ethylene propylene rubbers, one or more ethylene propylene diene rubbers, one or more crosslinked backbone unsaturated styrene terpolymers, or blends of any two or more thereof. In another embodiment, suitable stopper polymeric materials include, but are not limited to, one or more butadiene rubbers, one or more halogenated butyl rubbers (e.g., chloro butyl rubber or bromo butyl rubber), one or more crosslinked backbone unsaturated styrene terpolymers, or blends of any two or more thereof.

In another embodiment, stopper 38 is desirably manufactured from any suitable polymeric material with either a Young's modulus and/or a Shore A hardness in one of the ranges noted above in combination with a high Mooney viscosity, where the base polymer/rubber material's Mooney viscosity is determined before curing. A high Mooney rubber is defined as rubber having Mooney viscosity greater than about 40, or greater than about 45, or greater than about 50, or greater than about 55, or greater than about 60, or greater than about 65, or even greater than about 70. Here, as well as elsewhere in the specification and claims, individual numerical values can be combined to form additional, or even new/non-disclosed, numerical ranges.

Suitable high Mooney viscosity rubbers include, but are not limited to, one or more polybutadiene rubbers, one or more polyisobutylene, one or more polyisoprene rubbers, natural rubber, one or more ethylene propylene rubbers, one or more ethylene propylene diene rubbers, one or more styrene-butadiene rubbers, or blends of any two or more thereof.

In one embodiment, stopper 38 is manufactured from a polymeric material with a significantly lower coefficient of friction and contact area/pressure relative to a conventional rubber stopper. In one embodiment, the desired coefficient of friction of the afore-mentioned suitable polymer materials for stopper 38 of the present invention is between about 0.1 to about 0.98, or from about 0.11 to about 0.97, or from about 0.12 to about 0.96, or from about 0.13 to about 0.95, or from about 0.14 to about 0.94, or from about 0.15 to about 0.93, or from about 0.16 to about 0.92, or from about 0.17 to about 0.91, or from about 0.18 to about 0.90, or from about 0.19 to about 0.89, or from about 0.2 to about 0.88, or from about 0.21 to about 0.87, or from about 0.22 to about 0.86, or from about 0.23 to about 0.85, or from about 0.24 to about 0.84, or from about 0.25 to about 0.83, or from about 0.26 to about 0.82, or from about 0.27 to about 0.81, or from about 0.28 to about 0.80, or from about 0.29 to about 0.79, or from about 0.3 to about 0.78, or from about 0.31 to about 0.77, or from about 0.32 to about 0.76, or from about 0.33 to about 0.75, or from about 0.34 to about 0.74, or from about 0.35 to about 0.73, or from about 0.36 to about 0.72, or from about 0.37 to about 0.71, or from about 0.38 to about 0.70, or from about 0.39 to about 0.69, or from about 0.4 to about 0.68, or from about 0.41 to about 0.67, or from about 0.42 to about 0.66, or from about 0.43 to about 0.65, or from about 0.44 to about 0.64, or from about 0.45 to about 0.63, or from about 0.46 to about 0.62, or from about 0.47 to about 0.61, or from about 0.48 to about 0.60, or from about 0.49 to about 0.59, or from about 0.5 to about 0.58, or from about 0.51 to about 0.57, or from about 0.52 to about 0.56, or from about 0.53 to about 0.55, or even about 0.54. Here, as well as elsewhere in the specification and claims, individual numerical values can be combined to form additional, or even new/non-disclosed, numerical ranges. Accordingly, in this embodiment, stopper 38 can be used with a syringe 10 without silicone oil or other lubricants. In addition, the syringe barrel 12 may be made of the same material, a similar material, or even a completely different material from that used to form stopper 38.

With reference now to Figures 4-8, additional stopper body designs are shown in accordance with embodiments of the present invention.

Turning to Figure 4, Figure 4 discloses a stopper 600 having a laminated surface 602 thereon. As can be seen from Figure 4, stopper 600 has three ribs 604a, 604b, and 604c. Although stopper 600 has been shown with three ribs, ribs 604a, 604b, and 604c, the present invention is not limited thereto. Rather, as noted herein, any suitable number of ribs can be formed in stopper 600 as desired by the design parameters under consideration for stopper 600.

Continuing on further, stopper 600 further has a threaded portion 606, which in a non-limiting instance, can be formed in the shape of an Acme thread structure 606. As disclosed therein, stopper 600 can be any suitable size as determined by its length and width. In one non-limiting embodiment, stopper 600 is about 8 mm in length and has a width 608 (defined as OD_{STOPPER BODY}) at its widest point that is determined, per one non-limiting instance, by the compression ratio necessary to achieve the desired design parameters of the present invention. In the case where stopper 600 is about 8 mm in length, the radii of ribs 604a, 604b, and 604c are, in one non-limiting instance, about 5 mm or less. In another instance, each of ribs 604a, 604b, and 604c can independently each have their own rib radius so long as each different rib radius is about 5 mm or less. The body 610 of stopper 600 can be formed from any suitable polymeric material disclosed herein.

Continuing on with regard to stopper 600, in one non-limiting example, as is illustrated in Figure 4, the inner diameter 612 (ID_{STOPPER BODY}) of stopper 600 is designed to be greater than or equal to 85 percent (i.e., 0.85) of the outer diameter 608 (OD_{STOPPER BODY}) of stopper 600. That is, ID_{STOPPER BODY} ≥ 0.85 OD_{STOPPER BODY}. Additionally, in one non-limiting embodiment, the outer diameter 614 (OD_{THREAD}) of thread 606 is formed such that the outer diameter 614 (OD_{THREAD}) is less than or equal to 70 percent (i.e., 0.70) of the outer diameter 608 (OD_{STOPPER BODY}) of stopper 600. That is, OD_{THREAD} ≤ 0.7 OD_{STOPPER BODY}.

In one embodiment, stopper 600 has a flat front surface 618, but as would be appreciated by those of skill in the art could alternatively have any desired geometric design at the front surface thereof. As such, stopper 600 of Figure 4 has, in one non-limiting embodiment, three ribs, an Acme thread, a flat front, and is fully laminated. However, as is discussed herein, modifications to the design of stopper 600 are fully within the scope of the present invention and can include one or more exemplary non-limiting modifications such as a different number of ribs, one or more laminated ribs, one or more non-laminated ribs, a non-flat front surface, etc.

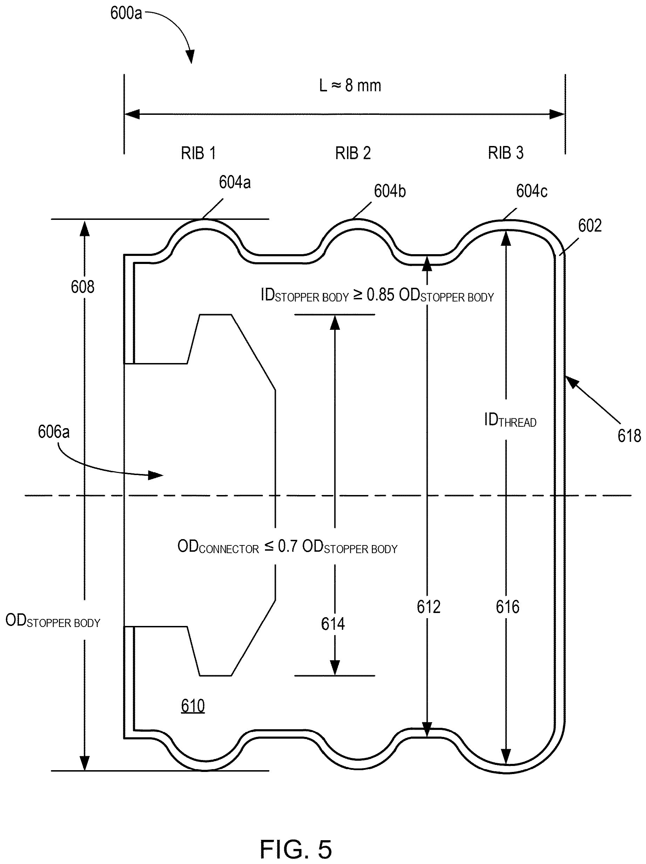

Turning to Figure 5, this figure represents a stopper design 600a where Acme thread structure 606 of stopper 600 has been replaced by a snap-in plunger rod connector 606a. Regarding the various geometric relationships of the stopper design of stopper 600a, these relationships are similar to that of stopper 600 where the inner diameter 612 (ID_{STOPPER BODY}) of stopper 600a is designed to be greater than or equal to 85 percent (i.e., 0.85) of the outer diameter 608 (OD_{STOPPER BODY}) of stopper 600a. That is, ID_{STOPPER BODY} ≥ 0.85 OD_{STOPPER BODY}. Additionally, in one non-limiting embodiment, the outer diameter 614 (OD_{CONNECTOR}) of connector 606a is formed such that the outer diameter 614 (OD_{CONNECTOR}) is less than or equal to 70 percent (i.e., 0.70) of the outer diameter 608 (OD_{STOPPER BODY}) of stopper 600a. That is, OD_{CONNECTOR} ≤ 0.7 OD_{STOPPER BODY}.

Although stopper 600a has been shown with three ribs, ribs 604a, 604b, and 604c, the present invention is not limited thereto. Rather, as noted herein, any suitable number of ribs can be formed in stopper 600a as desired by the design parameters under consideration for stopper 600a. Additionally, the body 610 of stopper 600a can be formed from any suitable polymeric material disclosed herein.

In one embodiment, stopper 600a has a flat front surface 618, but as would be appreciated by those of skill in the art could alternatively have any desired geometric design at the front surface thereof. As such, stopper 600a of Figure 5 has, in one non-limiting embodiment, three ribs, a snap-in plunger rod connector, a flat front, and is fully laminated. However, as is discussed herein, modifications to the design of stopper 600a are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600a is also about 8 mm in length and has a width 608 (defined as OD_{STOPPER BODY}) at its widest point that is determined, per one non-limiting instance, by the compression ratio necessary to achieve the desired design parameters of the present invention.

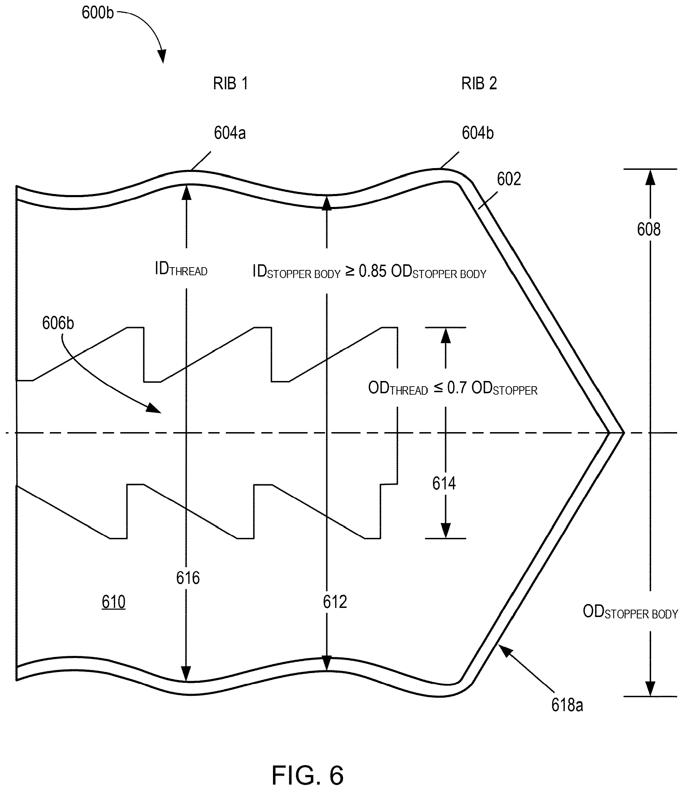

Turning to Figure 6, this figure represents a stopper design 600b where Acme thread structure 606 has been replaced by a buttress thread structure 606b and where stopper 600b only has two ribs 604a and 604b. Regarding the various geometric relationships of the stopper design of stopper 600b, these relationships are similar to that of stopper 600 where the inner diameter 612 (ID_{STOPPER BODY}) of stopper 600b is designed to be greater than or equal to 85 percent

(i.e., 0.85) of the outer diameter 608 (OD_{STOPPER BODY}) of stopper 600b. That is, ID_{STOPPER BODY} ≥ 0.85 OD_{STOPPER BODY}. Additionally, in one non-limiting embodiment, the outer diameter 614 (OD_{THREAD}) of thread 606b is formed such that the outer diameter 614 (OD_{THREAD}) is less than or equal to 70 percent (i.e., 0.70) of the outer diameter 608 (OD_{STOPPER BODY}) of stopper 600b. That is, OD_{THREADR} ≤ 0.7 OD_{STOPPER BODY}.

Although stopper 600b has been shown with two ribs, ribs 604a and 604b, the present invention is not limited thereto. Rather, as noted herein, any suitable number of ribs can be formed in stopper 600b as desired by the design parameters under consideration for stopper 600b. Additionally, the body 610 of stopper 600b can be formed from any suitable polymeric material disclosed herein.

In one embodiment, stopper 600b has a conically-shaped front surface 618a, but as would be appreciated by those of skill in the art could alternatively have any desired geometric design at the front surface thereof. As such, stopper 600b of Figure 6 has, in one non-limiting embodiment, two ribs, a buttress thread, a conically-shaped front, and is fully laminated. However, as is discussed herein, modifications to the design of stopper 600b are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600b has a width 608 (defined as OD_{STOPPER BODY}) at its widest point that is determined, per one non-limiting instance, by the compression ratio necessary to achieve the desired design parameters of the present invention.

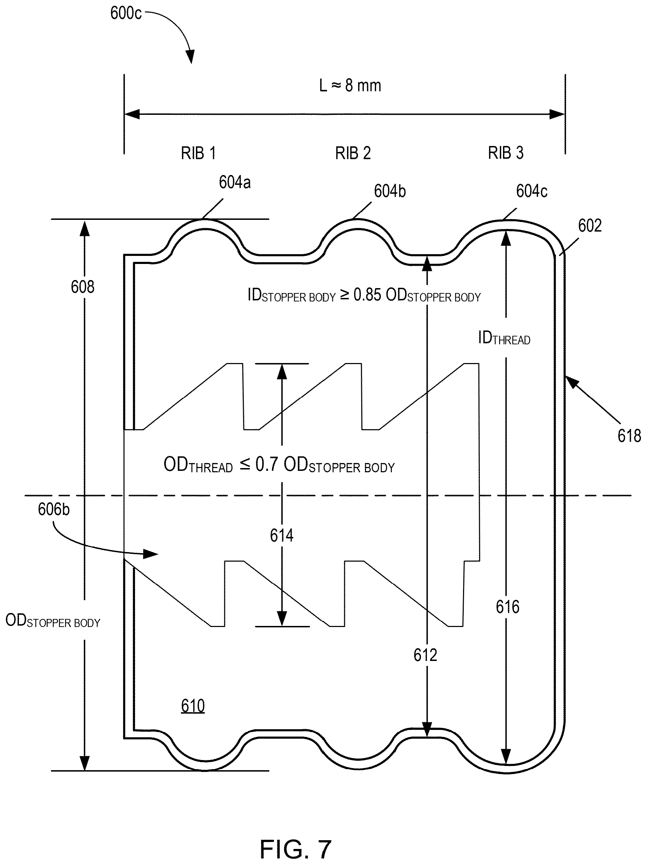

Turning to Figure 7, this figure represents a stopper design 600c where Acme thread structure 606 of stopper 600 has been replaced by a buttress thread structure 606b. Regarding the various geometric relationships of the stopper design of stopper 600c, these relationships are similar to that of stopper 600 where the inner diameter 612 (ID_{STOPPER BODY}) of stopper 600c is designed to be greater than or equal to 85 percent (i.e., 0.85) of the outer diameter 608 (OD_{STOPPER BODY}) of stopper 600c. That is, ID_{STOPPER BODY} ≥ 0.85 OD_{STOPPER BODY}. Additionally, in one non-limiting embodiment, the outer diameter 614 (OD_{THREAD}) of thread 606b is formed such that the outer diameter 614 (OD_{THREAD}) is less than or equal to 70 percent (i.e., 0.70) of the outer diameter 608 (OD_{STOPPER BODY}) of stopper 600c. That is, OD_{THREAD} ≤ 0.7 OD_{STOPPER BODY}.

Although stopper 600c of Figure 7 is illustrated having three ribs 604a, 604b, and 604c, the present invention is not limited thereto. Rather, as noted herein, any suitable number of ribs can be formed in stopper 600c as desired by the design parameters under consideration for stopper 600c. Additionally, the body 610 of stopper 600c can be formed from any suitable polymeric material disclosed herein.

In one embodiment, stopper 600c has a flat front surface 618, but as would be appreciated by those of skill in the art could alternatively have any desired geometric design at the front surface thereof. As such, stopper 600c of Figure 7 has, in one non-limiting embodiment, three ribs, a buttress thread, a flat front, and is fully laminated. However, as is discussed herein, modifications to the design of stopper 600c are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600c has a width 608 (defined as OD_{STOPPER BODY}) at its widest point that is determined, per one non-limiting instance, by the compression ratio necessary to achieve the desired design parameters of the present invention.

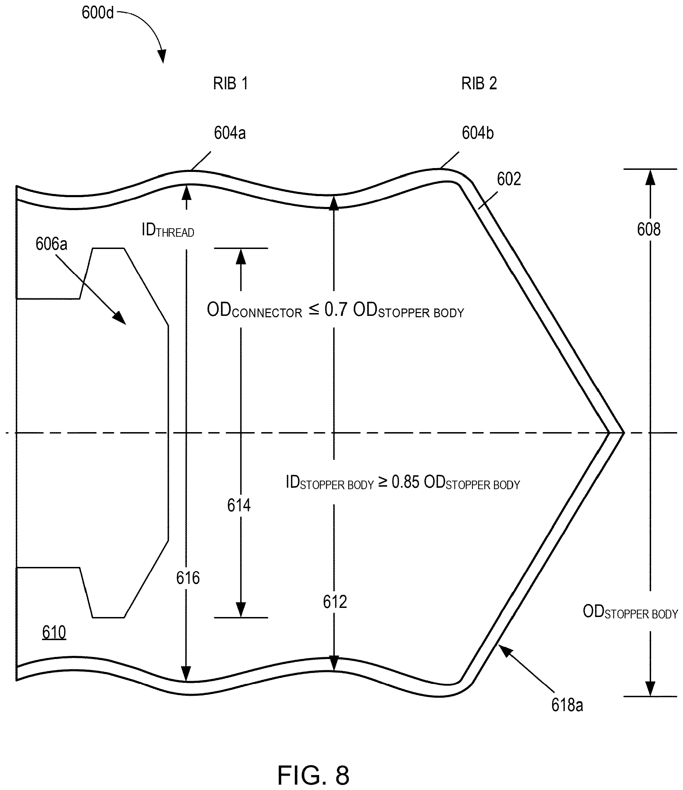

Turning to Figure 8, this figure represents a stopper design 600d where buttress thread structure 606b has been replaced by a snap-in plunger rod connector 606a and where stopper 600d only has two ribs 604a and 604b similar to stopper 600b. Regarding the various geometric relationships of the stopper design of stopper 600d, these relationships are similar to that of stopper 600b where the inner diameter 612 (ID_{STOPPER BODY}) of stopper 600d is designed to be greater than or equal to 85 percent (i.e., 0.85) of the outer diameter 608 (OD_{STOPPER BODY}) of stopper 600d. That is, ID _{STOPPER BODY} ≥ 0.85 OD_{STOPPER BODY}. Additionally, in one non-limiting embodiment, the outer diameter 614 (OD_{CONNECTOR}) of connector 606a is formed such that the outer diameter 614 (OD_{CONNECTOR}) is less than or equal to 70 percent (i.e., 0.70) of the outer diameter 608 (OD_{STOPPER BODY}) of stopper 600b. That is, OD_{CONNECTOR} ≤ 0.7 OD_{STOPPER BODY}.

Although stopper 600d has been shown with two ribs, ribs 604a and 604b, the present invention is not limited thereto. Rather, as noted herein, any suitable number of ribs can be formed in stopper 600d as desired by the design parameters under consideration for stopper 600b. Additionally, the body 610 of stopper 600d can be formed from any suitable polymeric material disclosed herein.

In one embodiment, stopper 600d has a conically-shaped front surface 618a, but as would be appreciated by those of skill in the art could alternatively have any desired geometric design at the front surface thereof. As such, stopper 600d of Figure 8 has, in one non-limiting embodiment, two ribs, a snap-in plunger rod connector, a conically-shaped front, and is fully laminated. However, as is discussed herein, modifications to the design of stopper 600d are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600d has a width 608 (defined as OD_{STOPPER BODY}) at its widest point that is determined, per one non-limiting instance, by the compression ratio necessary to achieve the desired design parameters of the present invention.

In one embodiment, due to the polymeric/rubber material that is used to form the stopper of the present invention (or any of the disclosed alternative stopper designs incorporated herein by reference), suitable sealing is accomplished by the contact pressure achieved by any one or more of a stopper in accordance with the present invention where such a stopper is formed from any one or more of the polymeric materials having one or more of the properties disclosed herein. That is, the suitable sealing properties of the stoppers of the present invention are achieved due to one or more of stopper geometry, the Young's modulus of the polymeric material used to form such a stopper, the Shore A hardness of the polymeric material used to form such a stopper, the coefficient of friction of the polymeric material used to form such a stopper, the indentation modulus of the laminate sealing surface, and/or the roughness of both the surface of the stopper and/or the inner surface of the barrel (or other container) that the stopper is designed to seal.

In one embodiment, the surface roughness of the glass, polymer, metal or another type of material that forms the surface to be sealed (such as a vial, syringe, or other type of container) by the stopper of the present invention is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In one embodiment, the surface roughness of the polymeric material that is used to form the stopper of the present invention is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In still another embodiment, if the surface to be sealed is partially, or fully, laminated the surface roughness of the laminated film, or the top most laminated film in the case where two or more films are used to provide an overall laminated coating, is independently in a range of about 0.0001 microns to about 12 microns, or from about 0.0002 microns to about 11.9 microns, or from about 0.0003 microns to about 11.8 microns, or from about 0.0004 microns to about 11.7 microns, or from about 0.0005 microns to about 11.6 microns, or from about 0.0006 microns to about 11.5 microns, or from about 0.0007 microns to about 11.4 microns, or from about 0.0008 microns to about 11.3 microns, or from about 0.0009 microns to about 11.2 microns, or from about 0.001 microns to about 11.1 microns, or from about 0.0011 microns to about 11 microns, or from about 0.0012 microns to about 10.9 microns, or from about 0.0013 microns to about 10.8 microns, or from about 0.0014 microns to about 10.7 microns, or from about 0.0015 microns to about 10.6 microns, or from about 0.0016 microns to about 10.5 microns, or from about 0.0017 microns to about 10.4 microns, or from about 0.0018 microns to about 10.3 microns, or from about 0.0019 microns to about 10.2 microns, or from about 0.002 microns to about 10.1 microns, or from about 0.0021 microns to about 10 microns, or from about 0.0022 microns to about 9.9 microns, or from about 0.0023 microns to about 9.8 microns, or from about 0.0024 microns to about 9.7 microns, or from about 0.0025 microns to about 9.6 microns, or from about 0.0026 microns to about 9.5 microns, or from about 0.0027 microns to about 9.4 microns, or from about 0.0028 microns to about 9.3 microns, or from about 0.0029 microns to about 9.2 microns, or from about 0.003 microns to about 9.1 microns, or from about 0.0031 microns to about 9 microns, or from about 0.0032 microns to about 8.9 microns, or from about 0.0033 microns to about 8.8 microns, or from about 0.0034 microns to about 8.7 microns, or from about 0.0035 microns to about 8.6 microns, or from about 0.0036 microns to about 8.5 microns, or from about 0.0037 microns to about 8.4 microns, or from about 0.0038 microns to about 8.3 microns, or from about 0.0039 microns to about 8.2 microns, or from about 0.004 microns to about 8.1 microns, or from about 0.0041 microns to about 8 microns, or from about 0.0042 microns to about 7.9 microns, or from about 0.0043 microns to about 7.8 microns, or from about 0.0044 microns to about 7.7 microns, or from about 0.0045 microns to about 7.6 microns, or from about 0.0046 microns to about 7.5 microns, or from about 0.0047 microns to about 7.4 microns, or from about 0.0048 microns to about 7.3 microns, or from about 0.0049 microns to about 7.2 microns, or from about 0.005 microns to about 7.1 microns, or from about 0.0051 microns to about 7 microns, or from about 0.0052 microns to about 6.9 microns, or from about 0.0053 microns to about 6.8 microns, or from about 0.0054 microns to about 6.7 microns, or from about 0.0055 microns to about 6.6 microns, or from about 0.0056 microns to about 6.5 microns, or from about 0.0057 microns to about 6.4 microns, or from about 0.0058 microns to about 6.3 microns, or from about 0.0059 microns to about 6.2 microns, or from about 0.006 microns to about 6.1 microns, or from about 0.0065 microns to about 6 microns, or from about 0.007 microns to about 5.9 microns, or from about 0.0075 microns to about 5.8 microns, or from about 0.008 microns to about 5.7 microns, or from about 0.0085 microns to about 5.6 microns, or from about 0.009 microns to about 5.5 microns, or from about 0.0095 microns to about 5.4 microns, or from about 0.01 microns to about 5.3 microns, or from about 0.015 microns to about 5.2 microns, or from about 0.02 microns to about 5.1 microns, or from about 0.025 microns to about 5 microns, or from about 0.03 microns to about 4.9 microns, or from about 0.035 microns to about 4.8 microns, or from about 0.04 microns to about 4.7 microns, or from about 0.045 microns to about 4.6 microns, or from about 0.05 microns to about 4.5 microns, or from about 0.055 microns to about 4.4 microns, or from about 0.06 microns to about 4.3 microns, or from about 0.065 microns to about 4.2 microns, or from about 0.07 microns to about 4.1 microns, or from about 0.075 microns to about 4 microns, or from about 0.08 microns to about 3.9 microns, or from about 0.085 microns to about 3.8 microns, or from about 0.09 microns to about 3.7 microns, or from about 0.095 microns to about 3.6 microns, or from about 0.1 microns to about 3.5 microns, or from about 0.15 microns to about 3.4 microns, or from about 0.2 microns to about 3.3 microns, or from about 0.25 microns to about 3.2 microns, or from about 0.3 microns to about 3.1 microns, or from about 0.35 microns to about 3 microns, or from about 0.4 microns to about 2.9 microns, or from about 0.45 microns to about 2.8 microns, or from about 0.5 microns to about 2.7 microns, or from about 0.55 microns to about 2.6 microns, or from about 0.6 microns to about 2.5 microns, or from about 0.65 microns to about 2.4 microns, or from about 0.7 microns to about 2.3 microns, or from about 0.75 microns to about 2.2 microns, or from about 0.8 microns to about 2.1 microns, or from about 0.85 microns to about 2 microns, or from about 0.9 microns to about 1.9 microns, or from about 0.95 microns to about 1.8 microns, or from about 1 micron to about 1.7 microns, or from about 1.1 microns to about 1.6 microns, or from about 1.2 microns to about 1.5 microns, or from about 1.3 microns to about 1.4 microns, or even about 1.35 microns as measured at 50X via optical interferometry. Here, as well as elsewhere in the specification and claims, individual numerical values can be combined to form additional, or even new/non-disclosed, numerical ranges.

In still another embodiment, if the stopper of the present invention is partially, or fully, laminated the surface roughness of the laminated film, or the top most laminated film in the case where two or more films are used to provide an overall laminated coating, is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In the embodiment where the syringe barrel is formed from glass, the compression ratio of the stopper versus the glass barrel inner diameter is from about 1 percent to about to about 10 percent, or from about 1.25 percent to about 9.75 percent, or from about 1.5 percent to about 9.5 percent, or from about 1.75 percent to about 9.25 percent, or from about 2 percent to about 9 percent, or from about 2.25 percent to about 8.75 percent, or from about 2.5 percent to about 8.5 percent, or from about 2.75 percent to about 8.25 percent, or from about 3 percent to about 8 percent, or from about 3.25 percent to about 7.75 percent, or from about 3.5 percent to about 7.5 percent, or from about 3.75 percent to about 7.25 percent, or from about 4 percent to about 7 percent, or from about 4.25 percent to about 6.75 percent, or from about 4.5 percent to about 6.5 percent, or from about 4.75 percent to about 6.25 percent, or from about 5 percent to about 6 percent, or from about 5.25 percent to about 5.75 percent, or even about 5.5 percent as determined from nominal dimensions meaning maximum outer diameter of the stopper in free state versus minimum sealing barrel inner diameter (consider in some instances that there may be a draft on the barrel inner diameter). Here, as well as elsewhere in the specification and claims, individual numerical values can be combined to form additional, or even new/non-disclosed, numerical ranges.

In one embodiment, the contact pressure generated at the sealing surface (be it glass, polymer, metal or another type of material that forms the surface to be sealed) by a stopper formed from a polymeric material in accordance with the present invention as measured within about a 0.01 mm width is more than about 90 percent of the indentation modulus, or yield strength, of a corresponding film of the polymeric material used to form such a stopper. As is known, the indentation modulus describes the elastic surface behavior of a material, in this case a polymeric material, during indentation. In another embodiment, the contact pressure generated at the sealing surface as measured within about a 0.01 mm width is more than about 91 percent, more than about 92 percent, more than about 93 percent, more than about 94 percent, more than about 95 percent, or even more than about 96 percent of the indentation modulus, or yield strength, of a corresponding film of the polymeric material used to form such a stopper. Here, as well as elsewhere in the specification and claims, individual numerical values can be combined to form additional, or even new/non-disclosed, numerical ranges. Alternatively, any of the contact pressure values noted above can be determined by comparing same to the contact pressure of a desired polymeric material at 50 percent elongation modulus.

In one embodiment, the polymeric/rubber material that is used to form a stopper of the present invention has a rubber substrate modulus in a range of about 4 MPa to about 13 MPa, or from about 4.25 MPa to about 12.75 MPa, or from about 4.5 MPa to about 12.5 MPa, or from about 4.75 MPa to about 12.25 MPa, or from about 5 MPa to about 12 MPa, or from about 5.25 MPa to about 11.75 MPa, or from about 5.5 MPa to about 11.5 MPa, or from about 5.75 MPa to about 11.25 MPa, or from about 6 MPa to about 11 MPa, or from about 6.25 MPa to about 10.75 MPa, or from about 6.5 MPa to about 10.5 MPa, or from about 6.75 MPa to about 10.25 MPa, or from about 7 MPa to about 10 MPa, or from about 7.25 MPa to about 9.75 MPa, or from about 7.5 MPa to about 9.5 MPa, or from about 7.75 MPa to about 9.25 MPa, or from about 8 MPa to about 9 MPa, or from about 8.25 MPa to about 8.75 MPa, or even about 8.5 MPa as measured at the limit of the stress versus strain of a molded part formed from a desired polymeric material without a laminated film thereon. Here, as well as elsewhere in the specification and claims, individual numerical values can be combined to form additional, or even new/non-disclosed, numerical ranges.

In one embodiment, the present invention uses various correlations between stopper geometry, roughness, indentation modulus of the surfaces, and/or modulus of substrate materials to achieve sealing (percolation threshold) that is independent of the surfaces energies of the sealing surfaces. As such, in one embodiment, the stoppers of the present invention are partially laminated and each stopper has a body formed from any polymeric material disclosed herein. In another embodiment, the stoppers of the present invention are partially laminated and each stopper has a body formed from any polymeric material that possesses, at a minimum, both a Young's modulus and Shore A hardness as disclosed herein, and that can be processed as described herein. In one instance, the body of any of the stoppers disclosed herein can be partially, or even fully, laminated with one or more fluoropolymer layers, and have one or more ribs (or even two or more ribs) laminated with the one or more fluoropolymer layers. In another embodiment, the stoppers of the present invention are fully laminated with all of the stopper body and ribs being laminated with one or more fluoropolymer layers. In one embodiment, the one or more fluoropolymer layers described herein may include a single layer of densified expanded polytetrafluoroethylene (ePTFE), or the one or more fluoropolymer layers may include a composite fluoropolymer film having a barrier layer and a porous layer, the barrier layer including densified ePTFE, polytetrafluoroethylene (PTFE), fluorinated ethylene propylene (FEP), polyethylene (e.g., UHMWPE), polypropylene (e.g., syndiotactic-PP), polyvinylidene fluoride, polyvinylfluoride, perfluoropropylevinylether, a perfluoroalkoxy polymer, polysulfone, and copolymers and combinations thereof. In still another embodiment, any high temperature material, such as a polymeric material, can be used to form the one or more lamination layers regardless of whether a stopper according to this embodiment is partially, or even fully, laminated. In still another embodiment, the stoppers of the present invention are laminated with one or more non-fluoropolymer materials. Suitable non-fluoropolymer materials for such lamination layers include, but are not limited to, UHMWPE, syndiotactic-polypropylene blends, and/or polysulfone.

The following is one non-limiting example of a material for the formation of stoppers in accordance with the present invention. It should be noted that the present invention is not limited solely to this one example but rather is to be broadly construed given the various parameters and properties set forth herein.

A stopper in accordance with the present invention is formed to have a stopper body with one rib, two ribs, or even three or more ribs thereon. The shape of such stopper is not critical and can be round, square, a composite shape, etc. The shape of the stopper is this example is such that the combination of the contact geometry-width, rubber modulus and roughness achieve along a continuous path along the sealing surface of any sealing element 90 percent or more of the indentation modulus of a corresponding film of the polymeric material used to form such a stopper (as defined in more detail above). The material used to form such a stopper is any rubber material described herein which achieves a Young's modulus of about 5 MPa to about 15 MPa, or even about 6 MPa to about 14 MPa (as defined in more detail above). In one embodiment, the material used to form the stopper of the present invention contains one or more suitable fillers, and can optionally even be a cross-linked polymeric material. Suitable filler materials for the polymeric materials include, but are not limited to, various silica compounds including silica compounds treated with one or more coupling agents (e.g., a silane coupling agent), single or multiple walled carbon nanotubes (CNT). Additionally, other suitable filler materials for the polymeric materials disclosed herein are known in the art and as such are omitted herein for the sake of brevity.

In another embodiment, the polymeric material that can be used to form a stopper in accordance with the present invention has a stress relaxation of between about 0 percent and about 35 percent as determined from initial contact max pressure. In still another embodiment, the polymeric material that is used to form a stopper of the present invention has a stress relaxation of between about 0 percent and about 30 percent, or between about 2.5 percent and about 27.5 percent, or between about 5 percent and about 25 percent, or between about 7.5 percent and about 22.5 percent, or between about 10 percent and about 20 percent, or between about 12.5 percent and about 17.5 percent, or even about 15 percent as determined from initial contact max pressure. Here, as well as elsewhere in the specification and claims, individual numerical values can be combined to form additional, or even new/non-disclosed, numerical ranges.

In one embodiment, the polymeric/rubber material for use in forming the stopper of the present invention is any crosslinked backbone unsaturated styrene terpolymer, or other polymer or rubber material, having a Shore A hardness in a range of about 60 to about 120, or from about 61 to about 119, or from about 62 to about 118, or from about 63 to about 117, or from about 64 to about 116, or from about 65 to about 115, or from about 66 to about 114, or from about 67 to about 113, or from about 68 to about 112, or from about 69 to about 111, or from about 70 to about 110, or from about 71 to about 109, or from about 72 to about 108, or from about 73 to about 107, or from about 74 to about 106, or from about 75 to about 105, or from about 76 to about 104, or from about 77 to about 103, or from about 78 to about 102, or from about 79 to about 101, or from about 80 to about 100, or from about 81 to about 99, or from about 82 to about 98, or from about 83 to about 97, or from about 84 to about 96, or from about 85 to about 95, or from about 86 to about 94, or from about 87 to about 93, or from about 88 to about 92, or from about 89 to about 91, or even about 90. In another embodiment, the polymeric material for use in forming the stopper of the present invention is any crosslinked backbone unsaturated styrene terpolymer having a Young's modulus in a range of about 5 MPa to about 15 MPa, or from about 5.1 MPa to about 14.9 MPa, or from about 5.2 MPa to about 14.8 MPa, or from about 5.3 MPa to about 14.7 MPa, or from about 5.4 MPa to about 14.6 MPa, or from about 5.5 MPa to about 14.5 MPa, or from about 5.6 MPa to about 14.4 MPa, or from about 5.7 MPa to about 9.3 MPa, or from about 5.8 MPa to about 9.2 MPa, or from about 5.9 MPa to about 9.1 MPa, or from about 6 MPa to about 9 MPa, or from about 6.1 MPa to about 8.9 MPa, or from about 6.2 MPa to about 8.8 MPa, or from about 6.3 MPa to about 8.7 MPa, or from about 6.4 MPa to about 8.6 MPa, or from about 6.5 MPa to about 8.5 MPa, or from about 6.6 MPa to about 8.4 MPa, or from about 6.7 MPa to about 8.3 MPa, or from about 6.8 MPa to about 8.2 MPa, or from about 6.9 MPa to about 8.1 MPa, or from about 7 MPa to about 8 MPa, or from about 7.1 MPa to about 7.9 MPa, or from about 7.2 MPa to about 7.8 MPa, or from about 7.3 MPa to about 7.7 MPa, or from about 7.4 MPa to about 7.6 MPa, or even about 7.5 MPa. In still another embodiment, the polymeric material for use in forming the stopper of the present invention is any suitable butyl rubber material having a Shore A hardness within one of the above ranges in conjunction with a Young's modulus within one of the above ranges. Here, as well as elsewhere in the specification and claims, individual numerical values can be combined to form additional, or even new/non-disclosed, numerical ranges.

In another embodiment, the polymeric material for use in forming the stopper of the present invention is any suitable butyl rubber material having a stress relaxation over a period of 24 hours better than about 20 percent at room temperature (RT), better than about 25 percent at RT, or even better than about 30 percent at RT. In still another embodiment, the polymeric material for use in forming the stopper of the present invention is any of the polymeric materials disclosed above that have a stress relaxation better than about 21 percent at room temperature (RT), better than about 22 percent at RT, better than about 23 percent at RT, better than about 24 percent at RT, better than about 25 percent at RT, better than about 26 percent at RT, better than about 27 percent at RT, better than about 28 percent at RT, better than about 29 percent at RT, or even better than about 30 percent at RT. Here, as well as elsewhere in the specification and claims, individual numerical values can be combined to form additional, or even new/non-disclosed, numerical ranges.

In still another embodiment, any of the polymeric materials disclosed for use in conjunction with the stoppers of the present invention can have any combination of two, three or more, or even four or more, of any of the various chemical and/or physical properties disclosed herein.

In one embodiment, the polymeric material for use in conjunction with the stoppers of the present invention are suitable butyl rubbers having a high Mooney viscosity, as defined above, that can be partially, or fully, laminated with one or more layers of PTFE, UHMWPE, or some other lamination material as detailed above which has a melting temperature that is within the rubber molding temperature parameters.

In one instance, a high Mooney viscosity bromo-butyl rubber composition is loaded with about 40 phr to about 110 phr reinforcing silica filler with a sulfur donor fast-accelerator type of curing package activated containing a metal oxide and stearic acid. In another instance, the loading range of this bromo-butyl rubber composition with the afore-mentioned silica filler is in a range of about 42 phr to about 108 phr, or from about 44 phr to about 106 phr, or from about 46 phr to about 104 phr, or from about 48 phr to about 102 phr, or from about 50 phr to about 100 phr, or from about 52 phr to about 98 phr, or from about 54 phr to about 96 phr, or from about 56 phr to about 94 phr, or from about 58 phr to about 92 phr, or from about 60 phr to about 90 phr, or from about 62 phr to about 88 phr, or from about 64 phr to about 86 phr, or from about 66 phr to about 84 phr, or from about 68 phr to about 82 phr, or from about 70 phr to about 80 phr, or from about 72 phr to about 78 phr, or from about 74 phr to about 76 phr, or even about 75 phr silica filler. As used herein, the term per hundred resin (phr) means the amount of an additive, resin, or some other component, by weight as calculated based on 100 parts by weight of base polymer, rubber, and/or resin. Here, as well as elsewhere in the specification and claims, individual numerical values can be combined to form additional, or even new/non-disclosed, numerical ranges.

Some alternative fillers are high reinforcement silica treated with a coupling agent such as a silane coupling agent, high surface area carbon based fillers such as furnace carbon blacks that comply with ASTM N110, N220, N330, N550, N660 standards, single wall and multi-wall carbon nano-tubes, graphene oxide nano-particles, phenolic and resol resin networks, and thermoplastics such as polypropylene. Additional rubber formulations for possible lamination with low melting temperature film (e.g., a suitable ultrahigh molecular weight polyethylene) are high Mooney viscosity bromo butyl rubber formulations having therein a high load (e.g., from about 60 phr to about 70 phr) reinforcing silica filler with a sulfur donor ultra-accelerator type of curing package (metal-organic complex) that has been activated with a metal oxide and a suitable carbamate-type secondary accelerator. In one embodiment the reinforcing silica filler is chosen from silica, sodium silicate, potassium silicate, calcium silicate, magnesium silicate, aluminum silicate. Additional suitable variants of this type of rubber formulation are those that utilize either a high reinforcement level (e.g., from about 60 phr to about 70 phr) of silica treated with a suitable coupling agent (e.g., a silane coupling agent), a dispersion polymer system, and/or a liquid rubber polymer additive (e.g., a suitable butadiene additive). Another variant of these type of rubber formulations utilize a high reinforcement level (e.g., from about 60 phr to 70 phr) of pre-treated silicates. The silica could be pretreated with a silane coupling agent, alkylammonium salts such as octadecylamine and octadecyltrimethylamine, and quaternary amines.

In one embodiment, a suitable filler according to the present invention is any of the filler materials disclosed herein that have a surface area greater than about 100 m²/g, or greater than about 110 m²/g, or greater than about 120 m²/g, or greater than about 130 m²/g, or greater than about 140 m²/g, or greater than about 150 m²/g, or greater than about 160 m²/g, or greater than about 170 m²/g, or greater than about 180 m²/g, or greater than about 190 m²/g, or greater than about 200 m²/g, or greater than about 210 m²/g, or greater than about 220 m²/g, or greater than about 230 m²/g, or greater than about 240 m²/g, or greater than about 250 m²/g, or greater than about 260 m²/g, or greater than about 270 m²/g, or greater than about 280 m²/g, or greater than about 290 m²/g, or even greater than about 300 m²/g. In another embodiment, a suitable filler according to the present invention is any of the filler materials disclosed herein that have a surface area greater than about 325 m²/g, or greater than about 350 m²/g, or greater than about 375 m²/g, or greater than about 400 m²/g, or greater than about 425 m²/g, or greater than about 450 m²/g, or greater than about 475 m²/g, or greater than about 500 m²/g, or greater than about 525 m²/g, or greater than about 550 m²/g, or greater than about 575 m²/g, or even greater than about 600 m²/g. Here, as well as elsewhere in the specification and claims, individual numerical values can be combined to form additional, or even new/non-disclosed, numerical ranges.

In an alternate embodiment the base polymeric material used in forming the stopper of the present invention is any suitable styrene-butadiene, high-cis butadiene, ethylene propylene diene terpolymer, brominated isobutylene paramethyl-styrene terpolymers, chloro or bromo isobutylene-isoprene co-polymer, one or more crosslinked backbone unsaturated styrene terpolymers, or any blend thereof having a Shore A hardness in a range of about 60 to about 100, or from about 61 to about 99, or from about 62 to about 98, or from about 63 to about 97, or from about 64 to about 96, or from about 65 to about 95, or from about 66 to about 94, or from about 67 to about 93, or from about 68 to about 92, or from about 69 to about 91, or from about 70 to about 90, or from about 71 to about 89, or from about 72 to about 88, or from about 73 to about 87, or from about 74 to about 86, or from about 75 to about 85, or from about 76 to about 84, or from about 77 to about 83, or from about 78 to about 82, or from about 79 to about 81, or even about 80. In an alternate embodiment one or more of these base polymers are blended with or without reinforcing fillers in combination with a curative package to obtain a Young's modulus in a range of about 5 MPa to about 15 MPa, or from about 5.1 MPa to about 14.9 MPa, or from about 5.2 MPa to about 14.8 MPa, or from about 5.3 MPa to about 14.7 MPa, or from about 5.4 MPa to about 14.6 MPa, or from about 5.5 MPa to about 14.5 MPa, or from about 5.6 MPa to about 14.4 MPa, or from about 5.7 MPa to about 14.3 MPa, or from about 5.8 MPa to about 14.2 MPa, or from about 5.9 MPa to about 14.1 MPa, or from about 6 MPa to about 14 MPa, or from about 6.1 MPa to about 13.9 MPa, or from about 6.2 MPa to about 13.8 MPa, or from about 6.3 MPa to about 13.7 MPa, or from about 6.4 MPa to about 13.6 MPa, or from about 6.5 MPa to about 13.5 MPa, or from about 6.6 MPa to about 13.4 MPa, or from about 6.7 MPa to about 13.3 MPa, or from about 6.8 MPa to about 13.2 MPa, or from about 6.9 MPa to about 13.1 MPa, or from about 7 MPa to about 13 MPa, or from about 7.1 MPa to about 12.9 MPa, or from about 7.2 MPa to about 12.8 MPa, or from about 7.3 MPa to about 12.7 MPa, or from about 7.4 MPa to about 12.6 MPa, or from about 7.5 MPa to about 12.5 MPa, or from about 7.6 MPa to about 12.4 MPa, or from about 7.7 MPa to about 12.3 MPa, or from about 7.8 MPa to about 12.2 MPa, or from about 7.9 MPa to about 12.1 MPa, or from about 8 MPa to about 12 MPa, or from about 8.1 MPa to about 11.9 MPa, or from about 8.2 MPa to about 11.8 MPa, or from about 8.3 MPa to about 11.7 MPa, or from about 8.4 MPa to about 11.6 MPa, or from about 8.5 MPa to about 11.5 MPa, or from about 8.6 MPa to about 11.4 MPa, or from about 8.7 MPa to about 11.3 MPa, or from about 8.8 MPa to about 11.2 MPa, or from about 8.9 MPa to about 11.1 MPa, or from about 9 MPa to about 11 MPa, or from about 9.1 MPa to about 10.9 MPa, or from about 9.2 MPa to about 10.8 MPa, or from about 9.3 MPa to about 10.7 MPa, or from about 9.4 MPa to about 10.6 MPa, or from about 9.5 MPa to about 10.5 MPa, or from about 9.6 MPa to about 10.4 MPa, or from about 9.7 MPa to about 10.3 MPa, or from about 9.8 MPa to about 10.2 MPa, or from about 9.9 MPa to about 10.1 MPa, or even about 10 MPa. Here, as well as elsewhere in the specification and claims, individual numerical values can be combined to form additional, or even new/non-disclosed, numerical ranges. In these embodiments acceptable cure packages could include sulfur donor fast-accelerator type of curing package activated containing a metal oxide and stearic acid, alternately, organic peroxides with or without type 1 and type 2 co-agents, and rubber maker sulfur in combination with thiazoles, sulfenamides, guanidines, and dithiocarbamates, individually or in unique combinations.

In one embodiment, the present invention relates to a polymer/rubber compound useful as a closure material in containers such as prefilled syringes and, more particularly, to a sealing component that can be processed at a relative low temperature to allow for the lamination with a low coefficient of friction film such as Ultra-High Molecular Weight Polyethylene (UHMWPE).

As noted above, in one embodiment the polymer/rubber material of the present invention has a coefficient of friction (CoF) below 1. Accordingly, in one embodiment the polymeric material of the present invention has a coefficient of friction between about 0.1 to about 0.98, or from about 0.11 to about 0.97, or from about 0.12 to about 0.96, or from about 0.13 to about 0.95, or from about 0.14 to about 0.94, or from about 0.15 to about 0.93, or from about 0.16 to about 0.92, or from about 0.17 to about 0.91, or from about 0.18 to about 0.90, or from about 0.19 to about 0.89, or from about 0.2 to about 0.88, or from about 0.21 to about 0.87, or from about 0.22 to about 0.86, or from about 0.23 to about 0.85, or from about 0.24 to about 0.84, or from about 0.25 to about 0.83, or from about 0.26 to about 0.82, or from about 0.27 to about 0.81, or from about 0.28 to about 0.80, or from about 0.29 to about 0.79, or from about 0.3 to about 0.78, or from about 0.31 to about 0.77, or from about 0.32 to about 0.76, or from about 0.33 to about 0.75, or from about 0.34 to about 0.74, or from about 0.35 to about 0.73, or from about 0.36 to about 0.72, or from about 0.37 to about 0.71, or from about 0.38 to about 0.70, or from about 0.39 to about 0.69, or from about 0.4 to about 0.68, or from about 0.41 to about 0.67, or from about 0.42 to about 0.66, or from about 0.43 to about 0.65, or from about 0.44 to about 0.64, or from about 0.45 to about 0.63, or from about 0.46 to about 0.62, or from about 0.47 to about 0.61, or from about 0.48 to about 0.60, or from about 0.49 to about 0.59, or from about 0.5 to about 0.58, or from about 0.51 to about 0.57, or from about 0.52 to about 0.56, or from about 0.53 to about 0.55, or even about 0.54. Here, as well as elsewhere in the specification and claims, individual numerical values can be combined to form additional, or even new/non-disclosed, numerical ranges.

In another embodiment, the one or more materials (e.g., UHMWPE) used to partially, or fully, laminate one or more portions of any polymer, or any object formed therefrom (e.g., the various stoppers disclosed herein), has a coefficient of friction of less than 1. Accordingly, in one embodiment the one or more materials used to partially, or fully, laminate one or more portions of any polymer, or any object formed therefrom, has a coefficient of friction of between about 0.1 to about 0.98, or from about 0.11 to about 0.97, or from about 0.12 to about 0.96, or from about 0.13 to about 0.95, or from about 0.14 to about 0.94, or from about 0.15 to about 0.93, or from about 0.16 to about 0.92, or from about 0.17 to about 0.91, or from about 0.18 to about 0.90, or from about 0.19 to about 0.89, or from about 0.2 to about 0.88, or from about 0.21 to about 0.87, or from about 0.22 to about 0.86, or from about 0.23 to about 0.85, or from about 0.24 to about 0.84, or from about 0.25 to about 0.83, or from about 0.26 to about 0.82, or from about 0.27 to about 0.81, or from about 0.28 to about 0.80, or from about 0.29 to about 0.79, or from about 0.3 to about 0.78, or from about 0.31 to about 0.77, or from about 0.32 to about 0.76, or from about 0.33 to about 0.75, or from about 0.34 to about 0.74, or from about 0.35 to about 0.73, or from about 0.36 to about 0.72, or from about 0.37 to about 0.71, or from about 0.38 to about 0.70, or from about 0.39 to about 0.69, or from about 0.4 to about 0.68, or from about 0.41 to about 0.67, or from about 0.42 to about 0.66, or from about 0.43 to about 0.65, or from about 0.44 to about 0.64, or from about 0.45 to about 0.63, or from about 0.46 to about 0.62, or from about 0.47 to about 0.61, or from about 0.48 to about 0.60, or from about 0.49 to about 0.59, or from about 0.5 to about 0.58, or from about 0.51 to about 0.57, or from about 0.52 to about 0.56, or from about 0.53 to about 0.55, or even about 0.54, regardless of the coefficient of friction of the underlying polymer/rubber material. Here, as well as elsewhere in the specification and claims, individual numerical values can be combined to form additional, or even new/non-disclosed, numerical ranges.

In still another embodiment, both the underlying polymer/rubber material that is to be partially, or fully, laminated as well as the one or more materials used to partially, or fully, laminate said polymer/rubber material have a coefficient of friction of less than 1. Accordingly, in one embodiment both of these materials, or sets of materials independently have a coefficient of friction of between about 0.1 to about 0.98, or from about 0.11 to about 0.97, or from about 0.12 to about 0.96, or from about 0.13 to about 0.95, or from about 0.14 to about 0.94, or from about 0.15 to about 0.93, or from about 0.16 to about 0.92, or from about 0.17 to about 0.91, or from about 0.18 to about 0.90, or from about 0.19 to about 0.89, or from about 0.2 to about 0.88, or from about 0.21 to about 0.87, or from about 0.22 to about 0.86, or from about 0.23 to about 0.85, or from about 0.24 to about 0.84, or from about 0.25 to about 0.83, or from about 0.26 to about 0.82, or from about 0.27 to about 0.81, or from about 0.28 to about 0.80, or from about 0.29 to about 0.79, or from about 0.3 to about 0.78, or from about 0.31 to about 0.77, or from about 0.32 to about 0.76, or from about 0.33 to about 0.75, or from about 0.34 to about 0.74, or from about 0.35 to about 0.73, or from about 0.36 to about 0.72, or from about 0.37 to about 0.71, or from about 0.38 to about 0.70, or from about 0.39 to about 0.69, or from about 0.4 to about 0.68, or from about 0.41 to about 0.67, or from about 0.42 to about 0.66, or from about 0.43 to about 0.65, or from about 0.44 to about 0.64, or from about 0.45 to about 0.63, or from about 0.46 to about 0.62, or from about 0.47 to about 0.61, or from about 0.48 to about 0.60, or from about 0.49 to about 0.59, or from about 0.5 to about 0.58, or from about 0.51 to about 0.57, or from about 0.52 to about 0.56, or from about 0.53 to about 0.55, or even about 0.54, regardless of the coefficient of friction of the underlying polymer/rubber material. Here, as well as elsewhere in the specification and claims, individual numerical values can be combined to form additional, or even new/non-disclosed, numerical ranges.

In another embodiment, the polymer/rubber material of the present invention can be any polymer/rubber material that can be processed using a process that is conducted at a temperature below about 130°C. In still another embodiment, the polymer/rubber material of the present invention can be any polymer/rubber material that is partially, or fully, laminated with one or more lamination compounds disclosed herein that can be processed using a process that is conducted at a temperature below about 130 °C. In still yet another embodiment, the processing processes disclosed herein are conducted at a temperature in the range of about 90 °C to about 130 °C, or from about 91 °C to about 129 °C, or from about 92 °C to about 128 °C, or from about 93 °C to about 127 °C, or from about 94 °C to about 126 °C, or from about 95 °C to about 125 °C, or from about 96 °C to about 124 °C, or from about 97 °C to about 123 °C, or from about 98 °C to about 122 °C, or from about 99 °C to about 121 °C, or from about 100 °C to about 120°C, or from about 101 °C to about 1q9 °C, or from about 102 °C to about 118 °C, or from about 103 °C to about 117 °C, or from about 104 °C to about 116 °C, or from about 105 °C to about 115 °C, or from about 106 °C to about 114 °C, or from about 107 °C to about 113 °C, or from about 108 °C to about 112 °C, or from about 109 °C to about 111 °C, or even about 110 °C. Here, as well as elsewhere in the specification and claims, individual numerical values can be combined to form additional, or even new/non-disclosed, numerical ranges. As used herein, the definition of processing completion is defined as an increase in ultimate torque response as measured by cure rheometer (ASTM 05289) of at least 400 percent within 10 minutes of isothermal conditioning at a platen temperature between about 90 °C and about 130 °C as further specified above. Thus, in one instance, the present invention involves a polymer/rubber formulation/compound/material that involves the use of innovative additives and a specific processing method/system that is both fast and activated at a lower temperature in the range of about 90 °C and about 130 °C as further specified above.

In one embodiment, the present invention is directed to a polymer/rubber composition comprising at least one brominated isobutylene paramethyl-styrene terpolymers (BIMSM) rubber with no unsaturation on the backbone of the polymer. Such polymers exhibit excellent barrier properties that make any stopper, or other closure or sealing device, ideal for a wide range of applications including, but not limited to, medical applications.

In still another embodiment, the present invention relates to a crosslinkable polymer/rubber compound featuring an ultra-accelerator activated by a metallic oxide for use in medical grade stopper applications. In one embodiment, the polymer/rubber composition of the present invention further comprises from about 40 phr to about 110 phr of at least one reinforcing non-staining halogenated isobutylene-isoprene copolymer and/or brominated isobutylene paramethyl-styrene terpolymers (BIMSM) of low to high Mooney viscosity. In another instance, the loading range of this at least one reinforcing non-staining halogenated isobutylene-isoprene copolymer and/or brominated isobutylene paramethyl-styrene terpolymers (BIMSM) of low to high Mooney viscosity in the polymer/rubber compositions of the present invention is in a range of about 42 phr to about 108 phr, or from about 44 phr to about 106 phr, or from about 46 phr to about 104 phr, or from about 48 phr to about 102 phr, or from about 50 phr to about 100 phr, or from about 52 phr to about 98 phr, or from about 54 phr to about 96 phr, or from about 56 phr to about 94 phr, or from about 58 phr to about 92 phr, or from about 60 phr to about 90 phr, or from about 62 phr to about 88 phr, or from about 64 phr to about 86 phr, or from about 66 phr to about 84 phr, or from about 68 phr to about 82 phr, or from about 70 phr to about 80 phr, or from about 72 phr to about 78 phr, or from about 74 phr to about 76 phr, or even about 75 phr non-staining halogenated isobutylene-isoprene copolymer and/or brominated isobutylene paramethyl-styrene terpolymers (BIMSM) of low to high Mooney viscosity. Here, as well as elsewhere in the specification and claims, individual numerical values can be combined to form additional, or even new/non-disclosed, numerical ranges.

Here a high Mooney viscosity is defined as noted above to a Mooney viscosity greater than about 40, or greater than about 45, or greater than about 50, or greater than about 55, or greater than about 60, or greater than about 65, or even greater than about 70. On the other hand, a low Mooney viscosity is defined to be less than about 40, or less than about 35, or less than about 30, or less than about 25, or less than about 20, or less than about 15, or even less than about 10. Here, as well as elsewhere in the specification and claims, individual numerical values can be combined to form additional, or even new/non-disclosed, numerical ranges.

In another embodiment, the polymer/rubber composition of the present invention further comprises from about 40 phr to about 110 phr (parts per hundred rubber) of at least one reinforcing non-staining halogenated isobutylene-isoprene copolymer and/or brominated isobutylene paramethyl-styrene terpolymers (BIMSM) of low to high Mooney viscosity as described above and further optionally comprises about 40 phr to about 80 phr of one or more one magnesium silicate clays (e.g., from a natural source) and/or about 10 phr to about 150 phr of one or more silica fillers that may be further modified with coupling agents, as previously described herein. The term "phr" as used herein, and according to conventional practice, refers to parts by weight of a respective material per 100 parts by weigh of rubber.

In another instance, the loading range of the one or more magnesium silicate clays in the polymer/rubber compositions of the present invention is in a range of about 42 phr to about 78 phr, or from about 44 phr to about 76 phr, or from about 46 phr to about 74 phr, or from about 48 phr to about 72 phr, or from about 50 phr to about 70 phr, or from about 52 phr to about 68 phr, or from about 54 phr to about 66 phr, or from about 56 phr to about 64 phr, or from about 58 phr to about 62 phr, or even about 60 phr magnesium silicate clay. Here, as well as elsewhere in the specification and claims, individual numerical values can be combined to form additional, or even new/non-disclosed, numerical ranges.

In still another instance, the loading range of the one or more silica fillers in the polymer/rubber compositions of the present invention is in a range of about 12 phr to about 148 phr, or from about 14 phr to about 146 phr, or from about 16 phr to about 144 phr, or from about 18 phr to about 142 phr, or from about 20 phr to about 140 phr, or from about 22 phr to about 138 phr, or from about 24 phr to about 136 phr, or from about 26 phr to about 134 phr, or from about 28 phr to about 132 phr, or from about 30 phr to about 130 phr, or from about 32 phr to about 128 phr, or from about 34 phr to about 126 phr, or from about 36 phr to about 124 phr, or from about 38 phr to about 122 phr, or from about 40 phr to about 120 phr, or from about 42 phr to about 118 phr, or from about 44 phr to about 116 phr, or from about 46 phr to about 114 phr, or from about 48 phr to about 112 phr, or from about 50 phr to about 110 phr, or from about 52 phr to about 108 phr, or from about 54 phr to about 106 phr, or from about 56 phr to about 104 phr, or from about 58 phr to about 102 phr, or from about 60 phr to about 100 phr, or from about 62 phr to about 98 phr, or from about 64 phr to about 96 phr, or from about 66 phr to about 94 phr, or from about 68 phr to about 92 phr, or from about 70 phr to about 90 phr, or from about 72 phr to about 88 phr, or from about 74 phr to about 86 phr, or from about 76 phr to about 84 phr, or from about 78 phr to about 82 phr, or even about 80 phr silica filler. Here, as well as elsewhere in the specification and claims, individual numerical values can be combined to form additional, or even new/non-disclosed, numerical ranges.

In one embodiment, the polymer/rubber materials of the present invention further comprises one or more suitable siliceous pigments that include, but are not limited to, pyrogenic and/or precipitated siliceous pigments (e.g., silica). In one embodiment, precipitated silica is used at an amount disclosed above and further have a BET surface area, as measured using nitrogen gas, in the range of about 40 m²/g to about 600 m²/g, or from about 45 m²/g to about 595 m²/g, or from about 50 m²/g to about 590 m²/g, or from about 55 m²/g to about 585 m²/g, or from about 60 m²/g to about 580 m²/g, or from about 65 m²/g to about 575 m²/g, or from about 70 m²/g to about 570 m²/g, or from about 75 m²/g to about 565 m²/g, or from about 80 m²/g to about 560 m²/g, or from about 85 m²/g to about 555 m²/g, or from about 90 m²/g to about 550 m²/g, or from about 95 m²/g to about 545 m²/g, or from about 100 m²/g to about 540 m²/g, or from about 105 m²/g to about 535 m²/g, or from about 110 m²/g to about 530 m²/g, or from about 115 m²/g to about 525 m²/g, or from about 120 m²/g to about 520 m²/g, or from about 125 m²/g to about 515 m²/g, or from about 130 m²/g to about 510 m²/g, or from about 135 m²/g to about 505 m²/g, or from about 140 m²/g to about 500 m²/g, or from about 145 m²/g to about 495 m²/g, or from about 150 m²/g to about 490 m²/g, or from about 155 m²/g to about 485 m²/g, or from about 160 m²/g to about 480 m²/g, or from about 165 m²/g to about 475 m²/g, or from about 170 m²/g to about 470 m²/g, or from about 175 m²/g to about 465 m²/g, or from about 180 m²/g to about 460 m²/g, or from about 185 m²/g to about 455 m²/g, or from about 190 m²/g to about 450 m²/g, or from about 195 m²/g to about 445 m²/g, or from about 200 m²/g to about 440 m²/g, or from about 205 m²/g to about 435 m²/g, or from about 210 m²/g to about 430 m²/g, or from about 215 m²/g to about 425 m²/g, or from about 220 m²/g to about 420 m²/g, or from about 225 m²/g to about 415 m²/g, or from about 230 m²/g to about 410 m²/g, or from about 235 m²/g to about 405 m²/g, or from about 240 m²/g to about 400 m²/g, or from about 245 m²/g to about 395 m²/g, or from about 250 m²/g to about 390 m²/g, or from about 255 m²/g to about 385 m²/g, or from about 260 m²/g to about 380 m²/g, or from about 265 m²/g to about 375 m²/g, or from about 270 m²/g to about 370 m²/g, or from about 275 m²/g to about 365 m²/g, or from about 280 m²/g to about 360 m²/g, or from about 285 m²/g to about 355 m²/g, or from about 290 m²/g to about 350 m²/g, or from about 295 m²/g to about 345 m²/g, or from about 300 m²/g to about 340 m²/g, or from about 305 m²/g to about 335 m²/g, or from about 310 m²/g to about 330 m²/g, or from about 315 m²/g to about 325 m²/g, or even about 320 m²/g. Here, as well as elsewhere in the specification and claims, individual numerical values can be combined to form additional, or even new/non-disclosed, numerical ranges. The BET method of measuring surface area is described in the journal of the American Chemical Society, volume 60 - page 304. The silica of the present invention can also be characterized by having a dibutylphthalate (DBP) absorption value in a range of about 100 to about 400, or even from about 150 to about 300. In still another embodiment, the DBP absorption value of the silica of the present invention is in the range of about 105 to about 395, or from about 110 to about 390, or from about 115 to about 385, or from about 120 to about 380, or from about 125 to about 375, or from about 130 to about 370, or from about 135 to about 365, or from about 140 to about 360, or from about 145 to about 355, or from about 150 to about 350, or from about 155 to about 345, or from about 160 to about 340, or from about 165 to about 335, or from about 170 to about 330, or from about 175 to about 325, or from about 180 to about 320, or from about 185 to about 325, or from about 190 to about 320, or from about 195 to about 315, or from about 200 to about 310, or from about 205 to about 305, or from about 210 to about 300, or from about 215 to about 295, or from about 220 to about 290, or from about 225 to about 285, or from about 230 to about 280, or from about 235 to about 275, or from about 240 to about 270, , or from about 245 to about 265, or from about 250 to about 260, or even about 255. Here, as well as elsewhere in the specification and claims, individual numerical values can be combined to form additional, or even new/non-disclosed, numerical ranges.

In still another embodiment, the silica used in conjunction with the present invention has an average ultimate particle size, for example, in the range of about 0.01 microns to about 0.05 micron as determined by the electron microscope, although the silica particles may be even smaller, or possibly larger, in size and as such the present invention is not limited solely to silica particles of the aforementioned particle sizes. In another embodiment, the silica has a particle size of about 0.011 microns to about 0.049 microns, or from about 0.012 microns to about 0.048 microns, or from about 0.013 microns to about 0.047 microns, or from about 0.014 microns to about 0.046 microns, or from about 0.015 microns to about 0.045 microns, or from about 0.016 microns to about 0.044 microns, or from about 0.017 microns to about 0.043 microns, or from about 0.018 microns to about 0.042 microns, or from about 0.019 microns to about 0.041 microns, or from about 0.02 microns to about 0.04 microns, or from about 0.021 microns to about 0.039 microns, or from about 0.022 microns to about 0.038 microns, or from about 0.023 microns to about 0.037 microns, or from about 0.024 microns to about 0.036 microns, or from about 0.025 microns to about 0.035 microns, or from about 0.026 microns to about 0.034 microns, or from about 0.027 microns to about 0.033 microns, or from about 0.028 microns to about 0.032 microns, or from about 0.029 microns to about 0.031 microns, or even about 0.03 microns. Here, as well as elsewhere in the specification and claims, individual numerical values can be combined to form additional, or even new/non-disclosed, numerical ranges.

In still yet another embodiment, the polymer/rubber material of the present invention can further comprises up to about 50 phr of one or more suitable processing oils such as mineral, paraffin, napthenic, silicone, vegetable, glycerol, elastomeric (liquid polymer) oils, as well as any suitable combinations of two or more thereof. In still another embodiment, the amount of the one or more processing oils as used herein is in the range of about 0 phr to about 50 phr, or from about 2.5 phr to about 47.5 phr, or from about 5 phr to about 45 phr, or from about 7.5 phr to about 42.5 phr, or from about 10 phr to about 40 phr, or from about 12.5 phr to about 37.5 phr, or from about 15 phr to about 35 phr, or from about 17.5 phr to about 32.5 phr, or from about 20 phr to about 30 phr, or from about 22.5 phr to about 27.5 phr, or even about 25 phr. Here, as well as elsewhere in the specification and claims, individual numerical values can be combined to form additional, or even new/non-disclosed, numerical ranges.

It is understood that the polymer/ rubber composition of the present invention can be compounded by methods generally known in the rubber compounding arts, such as mixing the various curing agents with various commonly used additive materials such as, for example, accelerators, curing aids (such as activators and retarders), processing additives (such as oils, resins including, but not limited to, tackifying resins and/or plasticizers), fillers, pigments, fatty acids, zinc oxide, waxes, antioxidants and/or antiozonants, and/or peptizing agents. As is known to those of skill in the compounding arts, such one or more additives mentioned above are selected and used in conventional amounts depending on the intended use of the processed material of the present invention.

Given the above, in one embodiment, the present invention is directed to a polymer material comprising: at least one polymer compound having a Young's modulus in the range of about 5 MPa to about 15 MPa and a Shore A hardness in the range of about 60 to about 100, wherein the polymer material is processed at a temperature in the range of about 90 °C to about 130 °C, wherein such processing is defined as an increase in ultimate torque response as measured by cure rheometer (ASTM 05289) of at least 400 percent within 10 minutes of isothermal conditioning at a platen temperature between about 90 °C and about 130 °C. In another embodiment, the polymer material of the present invention is formed from a polymer compound having a Young's modulus in the range of about 7.5 MPa to about 12.5 MPa and a Shore A hardness in the range of about 70 to about 90. In still another embodiment, the polymer material of the present invention is selected from one or more styrene terpolymers. In still another embodiment, the one or more styrene terpolymers are selected from one or more crosslinked backbone unsaturated styrene terpolymers. In still another embodiment, the one or more styrene terpolymers are selected from at least one brominated isobutylene paramethyl-styrene terpolymers (BIMSM) rubber with no unsaturation on the backbone of the polymer. In still another embodiment, the polymer material of the present invention further comprises from about 50 phr to about 100 phr of at least one non-staining halogenated isobutylene-isoprene copolymer and/or brominated isobutylene paramethyl-styrene terpolymers (BIMSM) of low to high Mooney viscosity. In still another embodiment, the polymer material of the present invention further comprises about 50 phr to about 70 phr of calcined magnesium silicate. In still another embodiment, the polymer material of the present invention further comprises about 10 phr to about 150 phr of at least one silica filler. In still another embodiment, the at least one silica filler is modified with at least one coupling agent. In still another embodiment, the polymer material of the present invention further comprises up to about 50 phr of at least one processing oil.

In another embodiment, the present invention is directed to a stopper comprising: a stopper body formed from any of the polymeric materials disclosed herein; and at least one sealing rib formed on at least one portion of the stopper body. In still another embodiment, the stopper body further comprises at least two sealing ribs formed thereon. In still another embodiment, the at least one of the sealing ribs is laminated with at least one lamination layer. In still another embodiment, all of the sealing ribs are laminated with at least one lamination layer. In still another embodiment, all of the sealing ribs and the stopper body are laminated with at least one lamination layer. In still another embodiment, the at least one lamination layer is formed from at least one compound selected form densified expanded polytetrafluoroethylene (ePTFE), polytetrafluoroethylene (PTFE), fluorinated ethylene propylene (FEP), a polyethylene such as a UHMWPE, polypropylene, polyvinylidene fluoride, polyvinylfluoride, perfluoropropylevinylether, a perfluoroalkoxy polymer, and copolymers and combinations thereof. In still another embodiment, the at least one lamination layer is formed from at least one layer of polytetrafluoroethylene (PTFE) or UHMWPE.

In still another embodiment, the present invention is directed to a syringe comprising: a syringe barrel; a plunger, wherein the plunger is designed to fit within the syringe barrel; and a stopper according to any of the embodiments disclosed herein and formed from any of the polymeric materials disclosed herein, wherein the stopper is adapted for attachment to one end of the plunger so as act as a seal for the syringe barrel.

In still another embodiment, the present invention is directed to a sealing device comprising: a sealing body formed from any of the polymeric materials disclosed herein; and at least one sealing structure and/or or sealing rib formed on at least one portion of the sealing body, that can be utilized to seal any type of medical device and/or other device needing an adequate sealing device.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure that are known or customarily practiced in the art to which this disclosure pertains and which fall within the limits of the appended claims.

## Claims

1. A polymer material comprising:
at least one polymer compound having a Young's modulus in the range of about 5 MPa to about 15 MPa and a Shore A hardness in the range of about 60 to about 100,
wherein the polymer material is processed at a temperature in the range of about 90 °C to about 130 °C.

2. The polymer material of claim 1, wherein the processing of the polymer material is defined as an increase in ultimate torque response as measured by cure rheometer (ASTM 05289) of at least 400 percent within 10 minutes of isothermal conditioning at a platen temperature between about 90 °C and about 130 °C.

3. The polymer material of any of claims 1 or 2, wherein at least one polymer compound having a Young's modulus in the range of about 7.5 MPa to about 12.5 MPa and a Shore A hardness in the range of about 70 to about 90.

4. The polymer material of any of claims 1 to 3, wherein the at least one polymer compound is selected from one or more styrene terpolymers, and optionally, the one or more styrene terpolymers are selected from one or more crosslinked backbone unsaturated styrene terpolymers, and further optionally, the one or more styrene terpolymers are selected from at least one brominated isobutylene paramethyl-styrene terpolymers (BIMSM) rubber with no unsaturation on the backbone of the polymer.

5. The polymer material of any of claims 1 to 4, wherein the polymer material further comprises from about 50 phr to about 100 phr of at least one non-staining halogenated isobutylene-isoprene copolymer and/or brominated isobutylene paramethyl-styrene terpolymers (BIMSM) of low to high Mooney viscosity.

6. The polymer material of any of claims 1 to 5, wherein the polymer material further comprises about 50 phr to about 70 phr of calcined magnesium silicate.

7. The polymer material of any of claims 1 to 6, wherein the polymer material further comprises about 10 phr to about 150 phr of at least one silica filler, and optionally, the at least one silica filler is modified with at least one coupling agent.

8. The polymer material of any of claims 1 to 7, wherein the polymer material further comprises up to about 50 phr of at least one processing oil.

9. A stopper comprising:
a stopper body formed from a polymeric material of any of claims 1 to 8; and
at least one sealing rib formed on at least one portion of the stopper body.

10. The stopper of claim 9, wherein the stopper body further comprises at least two sealing ribs formed thereon.

11. The stopper of any of claims 9 or 10, wherein at least one of the sealing ribs is laminated with at least one lamination layer.

12. The stopper of any of claims 9 to 11, wherein all of the sealing ribs are laminated with at least one lamination layer.

13. The stopper of any of claims 9 to 12, wherein all of the sealing ribs and the stopper body are laminated with at least one lamination layer.

14. The stopper of any of claims 9 to 13, wherein the at least one lamination layer is formed from at least one compound selected form a polyethylene such as a UHMWPE, copolymers thereof, and combinations thereof.

15. A syringe comprising:
a syringe barrel;
a plunger, wherein the plunger is designed to fit within the syringe barrel; and
a stopper according to any of claims 9 to 14, wherein the stopper is adapted for attachment to one end of the plunger so as act as a seal for the syringe barrel.

16. A sealing device comprising:
a sealing body formed from a polymeric material of any of claims 1 to 15; and
at least one sealing structure and/or or sealing rib formed on at least one portion of the sealing body.
